# EUROPEAN PATENT SPECIFICATION

(11) **EP 2 048 225 B1**
(45) Date of publication and mention of the grant of the patent: **29.12.2010**
(21) Application number: 07020026.6
(22) Date of filing: 12.10.2007
(51) Int. Cl.: C12N 5/00, C07K 16/28

(54) **Method and kit for rapid isolation of human Foxp3+treg cells**
Verfahren und Kit zur schnellen Isolierung humaner Foxp3-Treg-Zellen
Procédé et kit pour l'isolement rapide de cellules Foxp3+treg humaines

(43) Date of publication of application: 15.04.2009
(73) Proprietor: Max-Delbrück-Centrum für Molekulare Medizin (MDC), 13125 Berlin (DE)
(72) Inventor: Rötzschke, Olaf, 13125 Berlin (DE); Falk, Kirsten, 13125 Berlin (DE); Kleinewietfeld, Markus, 13125 Berlin (DE)
(74) Representative: Forstmeyer, Dietmar

(56) References cited:
- US-A- 6 017 719
- US-A1- 2005 164 387
- HARTIGAN-O'CONNOR ET AL: "Human CD4+ regulatory T cells express lower levels of the IL-7 receptor alpha chain (CD127), allowing consistent identification and sorting of live cells" JOURNAL OF IMMUNOLOGICAL METHODS, ELSEVIER SCIENCE PUBLISHERS B.V.,AMSTERDAM, NL, vol. 319, no. 1-2, 18 January 2007 (2007-01-18), pages 41-52, XP005835607 ISSN: 0022-1759
- RONCAROLO MARIA-GRAZIA ET AL: "Regulatory T-cell immunotherapy for tolerance to self antigens and alloantigens in humans" NATURE REVIEWS IMMUNOLOGY, vol. 7, no. 8, August 2007 (2007-08), pages 585-598, XP002469607 ISSN: 1474-1733(print) 1474-1741(ele
- LIU WEIHONG ET AL: "CD127 expression inversely correlates with FoxP3 and suppressive function of human CD4(+) T reg cells" JOURNAL OF EXPERIMENTAL MEDICINE, vol. 203, no. 7, July 2006 (2006-07), pages 1701-1711, XP002469608 ISSN: 0022-1007
- SEDDIKI N ET AL: "Expression of interleukin (IL)-2 and IL-7 receptors discriminantes between human regulatory and activated T cells" JOURNAL OF EXPERIMENTAL MEDICINE, TOKYO, JP, vol. 203, no. 7, 10 July 2006 (2006-07-10), pages 1693-1700, XP003008358 ISSN: 0022-1007
- HOFFMANN ET AL: "Isolation of CD4<+>CD25<+> Regulatory T Cells for Clinical Trials" BIOLOGY OF BLOOD AND MARROW TRANSPLANTATION, KLUGE CARDEN JENNINGS PUBLISHING, CHARLOTTESVILLE, VA, US, vol. 12, no. 3, March 2006 (2006-03), pages 267-274, XP005586844 ISSN: 1083-8791

## Description

The present invention relates to methods for isolating human forkhead box P3 (Foxp3+) CD4+CD25+ regulatory T cells (herein referred to as Foxp3+ Treg cells) from a sample containing peripheral blood mononuclear cells (PBMCs), a kit for isolating human Foxp3+ Treg cells, and the use of anti-CD49d antibody and anti-CD127 antibody for the isolation of human Foxp3+ Treg cells.

### Background Art

Regulatory T cells, or 'Tregs' are fundamental in controlling various immune responses in that Tregs can rapidly suppress the activity of other immune cells. In particular, Tregs are crucial for maintaining tolerance by downregulating undesired immune responses to self and non-self antigens (see, e.g. Fontenot, J. D. & Rudensky, A. Y. Nat Immunol 6, 331-7 (2005); Sakaguchi, S., Annu Rev Immunol 22, 531-62 (2004)). For instance, Treg defects have been discovered in patients with multiple sclerosis (MS), type I diabetes (T1D), psoriasis, myasthenia gravis (MG) and other autoimmune diseases (Baecher-Allan, C. & Hafler, D. A., Immunol Rev 212, 203-16 (2006)). Similar links may also exist for atopy and allergic diseases (Robinson, D. S., Larche, M. & Durham, S. R., J Clin Invest 114, 1389-97 (2004)). For all these diseases reports exist pointing to a reduced *in vitro* immune suppression of the patient's Treg cells. This has led to an increasing interest in the possibility of using Tregs in immunotherapy to treat or prevent chronic infections, autoimmune diseases, allergies and transplantation-related complications, such as graft rejection or graft-versus- host disease (GvHD) (For a review, see Roncarolo, M. G. & Battaglia, M., Nat Rev Immunol 7, 585-98 (2007)).

The characteristic marker of Tregs is Foxp3. Methods for the isolation of human Foxp3+ Treg cells are known. For instance, Hoffmann, P. et al. Biol Blood Marrow Transplant 12, 267-74 (2006) describe the isolation of CD4+CD25+ T cells with regulatory function from standard leukapheresis products by using a 2-step magnetic cell-separation protocol. The generated cell products contained on average 49.5% Foxp3+ Treg cells. Also, commercial kits, e.g. CD4+CD25+ Regulatory T Cell Isolation Kit from Miltenyi Biotec or Dynal^{®} CD4+CD25+ Treg Kit from Invitrogen are available.

All of the hitherto described methods for isolation of human Foxp3+ Treg cells employ positive selection of Foxp3+ Treg cells based on cell surface markers of Tregs (see, e.g. Seddiki, N. et al., J Exp Med 203, 1693-700 (2006)). That is, the Foxp3+ Treg cells are isolated by using antibodies for Treg associated cell surface markers, mostly CD25. Yet most cell surface markers of Tregs, such as CD4, CD25 and Foxp3, are not restricted to Tregs. For instance, the commonly employed CD25 is also expressed by effector and memory CD4+ T cells (see, e.g. Baecher-Allan, C., Brown, J. A., Freeman, G. J. & Hafler, D. A., J Immunol 167, 1245-53 (2001)). Consequently, these positive selection methods do not permit the isolation of a uniform population that accounts for most of the Foxp3+ Treg cells as outlined above; Hoffmann, P. et al. obtained on average 49.5% Foxp3+ Treg cells.

Also, application of Foxp3+ Treg cells, that have been isolated by positive selection based on cell surface markers of Tregs, in cellular therapy poses severe problems. First, isolation of Foxp3+ Treg cells based on cell surface markers, e.g. CD25, leads to more or less severe contaminations of the Foxp3+ Treg cell population with other cells, e.g. CD4+ effector cells which represent the main target of Treg suppression. Accordingly, there is a high risk if such positively selected Foxp3+ Treg cells were to be applied in cellular therapy as it might lead to a potentially fatal activation of the immune system of the patient treated. Such a fatal immune response was recently documented in the failure of the 'Tegenero' trials (Suntharalingam, G. et al., N Engl J Med 355, 1018-28 (2006)). Second, positively selected Foxp3+ Treg cells which have been tagged by an antibody may exhibit impaired function. For cells tagged by an antibody are potentially preactivated, may suffer complement- or cell-mediated depletion or exhibit altered homing and migration patterns. Accordingly, Foxp3+ Treg cells targeted by antibodies during their isolation are undesirable not only for safety reasons.

As discussed, the methods and kits described above show major disadvantages with respect to isolating Foxp3+ Treg cells. Additionally, so far no method exists that allows to access Foxp3+ Treg cells by negative selection, i.e. leaving the Foxp3+ Treg cells label/antibody-free. It is therefore an object of the present invention to provide a method which avoids the above mentioned disadvantages of the prior art. Additionally, it is a further object of the present invention to provide a kit for the isolation of Foxp3+ Treg cells.

### Description of the invention

The present invention is based on results about the correlation of specific cell surface markers of Foxp3+ Treg cells and cell surface markers of non-regulatory CD4+ T cells. In particular the cell surface markers CD127 and CD49d were examined. During the experiments in the context of the present invention, it could be shown that the surface markers CD127 and CD49d are absent in most Foxp3+ Treg cells. In the context of the present invention, it could be shown that untouched human Foxp3+ Treg cells can be isolated in a single depletion step by the combined use of antibodies against CD127 and CD49d with great purity, e.g. a purity of >90% (i.e. Foxp3+ Treg cells constitute more than 90% of the cells in the isolated cell product). Additionally, the suppressor activity of the isolated Foxp3+ Treg cells was confirmed by experiments in the context of the present invention. The isolated Foxp3+ Treg cells strongly inhibited mixed lypmphocyte reactions (MLR) *in vitro* and prevented the fatal attack of transferred human PBMC *in vivo* in a GvHD model based on Rag2-/- γc-/- mice.

In a first aspect of the present invention, one of its objects is solved by a method for isolating human Foxp3+ Treg cells from a sample containing peripheral blood mononuclear cells, or 'PBMCs', the method comprising the steps of:
(a) treating the sample with an anti-CD127 antibody and an anti-CD49d antibody;
(b) separating Foxp3+ Treg cells.

The term "treating the sample" as used in the present invention shall especially imply that the cells contained in the sample are brought into direct physical contact with the antibodies in a way that the antibodies can interact with the targeted cells. In other words, in the method according to the present invention the peripheral blood mononuclear cells PBMCs are contacted with an anti-CD127 antibody and an anti-CD49d antibody and Foxp3+ Treg cells are separated.

The term "separating" as used in the present invention refers to the removal by physical means either of one cell type, e.g. Foxp3+ Treg cells, from other cell types, e.g. CD4+ effector cells, or of all non-regulatory CD4+ T cells from Foxp3+ Treg cells. For a more detailed description of separation techniques it is referred to P.T. Sharpe, Methods of Cell Separation, Laboratory Techniques in Biochemistry and Molecular Biology, Vol. 18, ELSEVIER (1988), and D. Fisher, G. E. Francis, D. Rickwood (Eds.), Cell Separation: A Practical Approach, Oxford University Press (1999).

As has been outlined above and in other terms, the invention solves the recited technical problem by a simple, highly reliable and reproducible method for the isolation of Foxp3+ Treg cells. In particular, as mentioned above, prior art methods rely on cell surface markers of Foxp3+ Treg cells. However, as explained, said methods only allowed the isolation of Foxp3+ Treg cells that were labelled, namely tagged by an antibody against one of the cell surface markers of the Foxp3+ Treg cells. Furthermore, said methods required at least two isolation steps: (i) depletion of non-CD4 cells; (ii) isolation of CD25+ cells. In contrast to this, the method of the present invention first of all allows the isolation of Foxp3+ Treg cells leaving said Foxp3+ Treg cells label-free, or 'untouched'. Additionally, the Foxp3+ Treg cells can be isolated in a single step. Thus, isolation of Foxp3+ Treg cells using the method according to the present invention is faster, easier and above all more effective with regard to the isolation of a uniform population that accounts for most of the Foxp3+ Treg cells contained in the sample. Finally, the method of the present invention can be automated, therefore further augmenting easy applicability of the method.

The most encouraging result obtained in accordance with the present invention was the finding that untouched Foxp3+ Treg cells can be obtained with high purity by the combined, i.e. sequential or simultaneous, use, of anti-CD49d antibody and anti-CD127 antibody, which target two cell surface markers, namely CD127 and CD49d, inversely correlated with Foxp3 expression, in the method according to the present invention. The isolated Foxp3+ Treg cells obtained by the method according to the present invention are fully functional, demonstrated by their capacity to inhibit mixed lymphocyte reactions *in vitro* and to prevent lethal xeno-GvHD responses *in vivo*. So far, contamination of isolated Foxp3+ Treg cells with non-regulatory CD4+ cells, e.g. effector and memory CD4+ cells, was the major drawback in isolation methods for Foxp3+ Treg cells. A considerable amount of non-regulatory CD4+ cells is present in Foxp3+ Treg cell populations isolated by positive selection based on cell surface markers of Tregs. For example, in the Foxp3+ Treg cell populations isolated by Hoffmann, P. et al. Biol Blood Marrow Transplant 12, 267-74 (2006) using positive selection on average more than 50% of the cells in these populations were non-regulatory CD4+ cells. Naturally, such considerable amounts of undesired non-regulatory CD4+ cells hamper the use of such Foxp3+ Treg cell populations, for example, in immunotherapy as mentioned above.

Preferably, in the method of the present invention steps (a) and (b) can be carried out simultaneously.

The advantage of carrying out steps (a) and (b) simultaneously lies in that the isolation of Foxp3+ Treg cells is less laborious, simple, fast and also more cost effective.

Further, in the method according to the present invention, step (a) can additionally comprise separation of non CD4+ T cells from PBMCs.

The advantage of additionally separating non CD4+ T cells from PBMCs lies in that non-regulatory CD4+ T cells are removed with higher efficiency and, as a result, the purity of the obtained cell product is higher.

In the method according to the present invention, non CD4+ T cells can be separated from PBMCs by negative selection using an anti-CD4 antibody.

The term "negative selection" as used in the present invention means that unwanted cells are removed from the repertoire of cells by labelling/capturing said unwanted cells, while leaving the cells of interest (label-)free.

Further, in the method according to the present invention, one or more antibody/antibodies that allow for the specific depletion of non CD4+ T cells from PBMCs can be used for the separation of non CD4+ T cells from PBMCs.

The advantage of separating non CD4+ T cells from PBMCs by depletion lies in that CD4+ T cells remain clean and untouched.

Preferred according to the present invention is a method, wherein the anibody/antibodies used for the specific depletion of non CD4+ T cells from PBMCs can be selected from the group comprising anti-CD8 antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody, or mixtures.

Preferred according to the present invention, anti-CD14 antibody, anti-CD15 antibody, and/or anti-CD16 antibody can be used for the specific depletion of non CD4+ T cells from PBMCs.

The advantage of using anti-CD14 antibody, anti-CD15 antibody, and/or anti-CD16 antibody for the specific depletion of non CD4+ T cells is that this subset of antibodies is small compared to some commercially available subsets.

Further preferred according to the present invention's method, anti-CD14 antibody and/or anti-CD16 antibody can be used for the specific depletion of non CD4+ T cells from PBMCs.

Preferred according to the present invention is further a method, wherein at least one of the antibodies used in step (a) is labelled or immobilized.

The term "labelled" as used in the present invention means that a molecule, e.g. an antibody, is conjugated to a label. Many different labels that can be conjugated to an antibody are known to the skilled artisan. For example, radioisotopes, e.g. ³²P, ³⁵S or ³H; fluorescence or luminescence markers, e.g. fluorescein (FITC), rhodamine, texas red, phycoerythrin (PE), allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA); antibodies or antibody fragments, e.g. F(ab)2 fragment; affinity labels, e.g. biotin, avidin, agarose, bone morphogenetic protein (BMP), matrix bound, haptens; and enzymes or enzyme substrates, e.g. alkaline phosphatase (AP) and horseradish peroxidase (HRP).

The term "immobilized" as used in the present invention refers to any support to which an antibody can be linked to while retaining its activity. Preferably, the support may be the surface of a matrix, e.g. a nylon matrix; a microtiter plate or a similar solid plastic support; beads, e.g. agarose or magentic beads. Immobilized antibodies are for example described in US 4,615,985 and in references cited therein.

The advantage of using labelled or immobilized antibodies compared to unlabelled antibodies is that labelled or immobilized antibodies can be easier used with standard equipment and also an adaptation to standard isolation techniques is facilitated.

Preferred according to the present invention, at least one antibody used in step (a) is immobilized.

Preferably, in the present invention's method, at least one antibody used in step (a) is immobilized on a nylon matrix.

The advantage of immobilizing an antibody on a nylon matrix lies in that immobilization on nylon matrices is very efficient and allows a flexible, easy, fast, simple and inexpensive column-based isolation of cells. Immobilisation on a nylon matrix is, for example, described in US 4,615,985.

Further preferred according to the present invention's method, the antibodies used in step (a) can be uniformly labelled.

The advantage of uniformly labelling the antibodies lies in that the antibodies can be detected all at once.

Preferably, in the present invention's method, the label can be selected from the group comprising isotopes, fluorescence or luminescence markers, antibodies or antibody fragments, affinity labels, and enzymes or enzyme substrates.

Preferred according to the present invention's method, the anti-CD127 antibody can be labelled with biotin, fluorescein (FITC) or phycoerythrin (PE).

Preferred according to the present invention's method, the anti-CD49d antibody can be labelled with biotin, fluorescein (FITC) or phycoerythrin (PE).

Preferred according to the present invention's method, step (b) can be carried out using centrifugation, particularly, density gradient centrifugation, cell elutriation, magnetic separation, fluorescence activated cell sorting, immunological separation, adhesion, complement lysis or flow cytometry.

Preferred according to the present invention's method, step (b) can be carried out using magnetic cell separation, fluorescence activated cell sorting, or a column-based immunological separation.

The term "column-based immunological separation" refers to a way of sorting cells, where antibodies employed in the method according to the invention can be attached to resins of chromatography columns and used to bind a cell that possesses an antigen recognized by the specific antibody.

Preferred according to the present invention's method, an anti-CD45RO antibody can be used as an additional antibody in step (a), and the isolated Foxp3+ Treg cells are CD45RA+ T cells.

The advantage of using an anti-CD45RO antibody as an additional antibody in step (a) lies in that a specific subset of Foxp3+ Treg cells, namely CD45RA+ T cells can be isolated in a highly efficient manner and a very high purity.

Preferred according to the present invention's method, an anti-CD45RA antibody can be used as an additional antibody in step (a), and the isolated Foxp3+ Treg cells are CD45RO+ T cells.

The advantage of using an anti-CD45RA antibody as an additional antibody in step (a) lies in that a specific subset of Foxp3+ Treg cells, namely CD45RO+ T cells can be isolated in a highly efficient manner and a very high purity.

In a second aspect of the present invention, one of its objects is solved by a kit for isolating human Foxp3+ Treg cells, comprising an anti-CD49d antibody and an anti-CD127 antibody.

Preferably, the kit according to the present invention can additionally comprise one or more antibody/antibodies selected from the group comprising anti-CD8 antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody.

Further, the kit of the present invention can contain at least one antibody that is immobilized.

Moreover, the kit according to the invention can contain at least one antibody that is labelled.

Preferably, the kit of the present invention can contain antibodies that are uniformly labelled.

Preferred the kit according to the invention can contain labelled antibodies, wherein the label can be selected from the group comprising isotopes, fluorescence or luminescence markers, antibodies or antibody fragments, affinity labels, and enzymes or enzyme substrates.

Preferably, the kit can contain an anti-CD127 antibody that can be labelled with biotin, FITC, or PE and an anti-CD49d antibody that can be labelled with biotin, FITC, or PE.

In a third aspect of the present invention, one of its objects is solved by a use of an anti-CD49d antibody and an anti-CD127 antibody or of a kit according to the invention for the isolation of human Foxp3+ Treg cells.

Preferably, the use according to the invention can be characterized in that separation of the human Foxp3+ Treg cells can be achieved by separating CD49d+CD127+ PBMCs including non-regulatory CD4+ T cells from unlabelled Foxp3+ Treg cells via centrifugation, cell elutriation, magnetic separation, fluorescence activated cell sorting, immunological separation, adhesion, complement lysis or flow cytometry.

Further, the use according to the invention can be characterized in that depletion of non CD4+ T cells from PBMCs can be carried out using at least one antibody selected from the group comprising anti-CD8 antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody.

### Figures

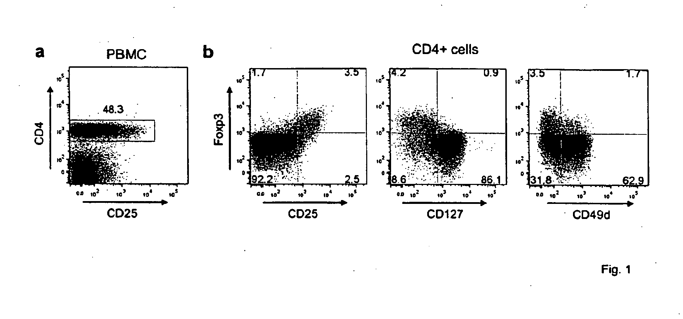

**Fig. 1****:** Inverse correlation of CD127 and CD49d with Foxp3 expression. Human PBMC were stained for CD4, CD25, CD127, CD49d and Foxp3 and analyzed by FACS. **a.** Double staining of human PBMC for CD4 and CD25. Percentage of CD4+ cells of total PBMC is indicated. **b.** Correlation of CD25, CD127 and CD49d with Foxp3 expression. Co-staining of Foxp3 with CD25 (left panel), CD127 (middle panel and CD49d is shown for CD4+ cells gated according to Fig. 1a. Numbers indicate percentage of cells in each quadrant.

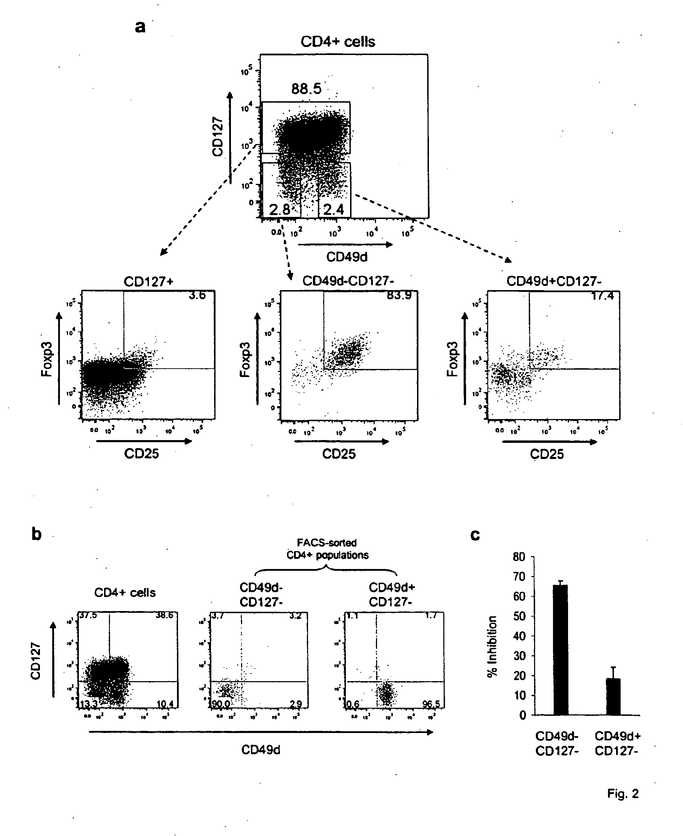

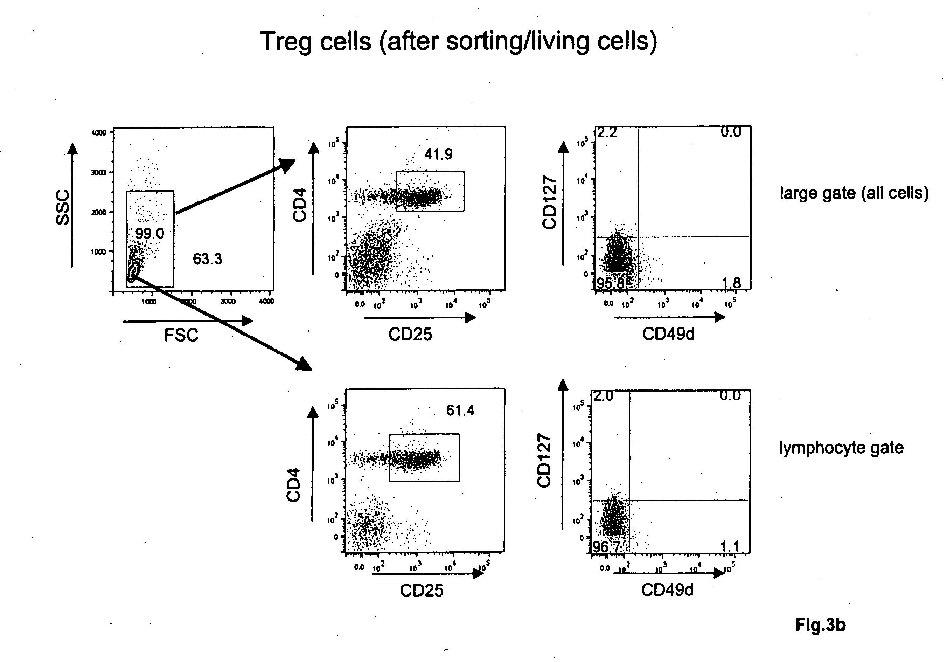

**Fig. 2****:** CD49d discriminates Foxp3+ Treg cells from Foxp3-CD127- cells. **a.** Foxp3 expression in CD127/CD49d subsets of CD4+ T cells. Human PBMC were stained for CD4, CD25, CD127, CD49d and Foxp3 and gated for CD4+ cells. Upper panel: co-staining of CD49d and CD127. Gates and percentages of the three major populations are indicated. Lower panels: Co-staining of CD25 and Foxp3 is shown for CD127+ (left panel), CD49d-CD127- (middle panel) and CD49d+CD127- cells (right panel). Percentages represent the number of CD25+Foxp3+ Treg cells in the indicated quadrant. **b.** Isolation of CD49d-CD127- and CD49d+CD127- cells by FACS sorting. Untouched CD4+ cells isolated from PBMC with commercial MACS-depletion kit were stained with α-CD49d and α-CD127 (left panel) and sorted by FACS into the CD49d-CD127- (middle panel) and the CD49d+CD127- subset (right panel). Numbers indicate the fraction of cells in each quadrant. **c.** Suppressive capacity. Inhibition of the proliferative response by the two isolated cell subsets was determined in a FACS based suppression assay as shown in Fig. 3b. Proliferation of CD4+ cells was induced by α-CD3 antibodies, suppressor cells were added at a ratio of 1:2. Suppression is expressed as '% inhibition' and was calculated by the fraction of dividing cells in reference to number of dividing α-CD3 stimulated CD4+ cells.

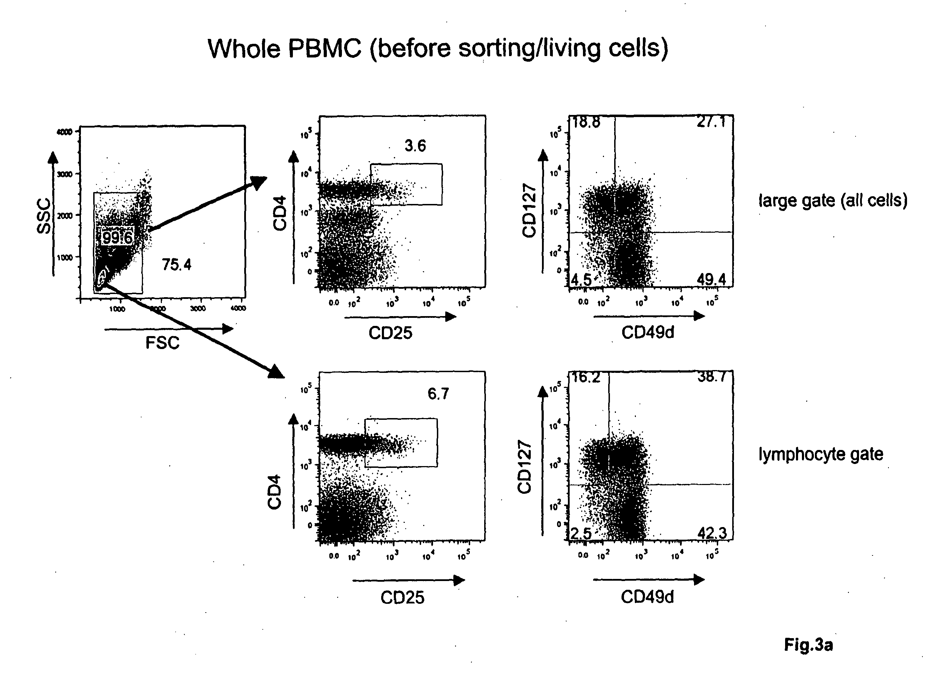

**Fig.3****:** Isolation of Foxp3 Treg cells by magnetic cell sorting. **a.** FACS profile for the starting population of PBMC from a healthy donor isolated by ficoll density gradient centrifugation. Left panel: FACS profile for the SSC vs. FSC. Right panels: FACS profiles for the populations gated on all cells (upper row) or on lymphocytes (lower row) indicated by the arrows for CD4 vs. CD25 or CD127 vs. CD49d. **b.** FACS profile for the bead negative population after depletion for CD127 and CD49d by the MACS system. Left panel: FACS profile of cells like in Fig.1a. Right panels: FACS profile for the expression of CD4 vs. CD25 and CD127 vs. CD49d for the indicated gates like in Fig.3a. Numbers indicate the percentage of cells in the corresponding quadrant.

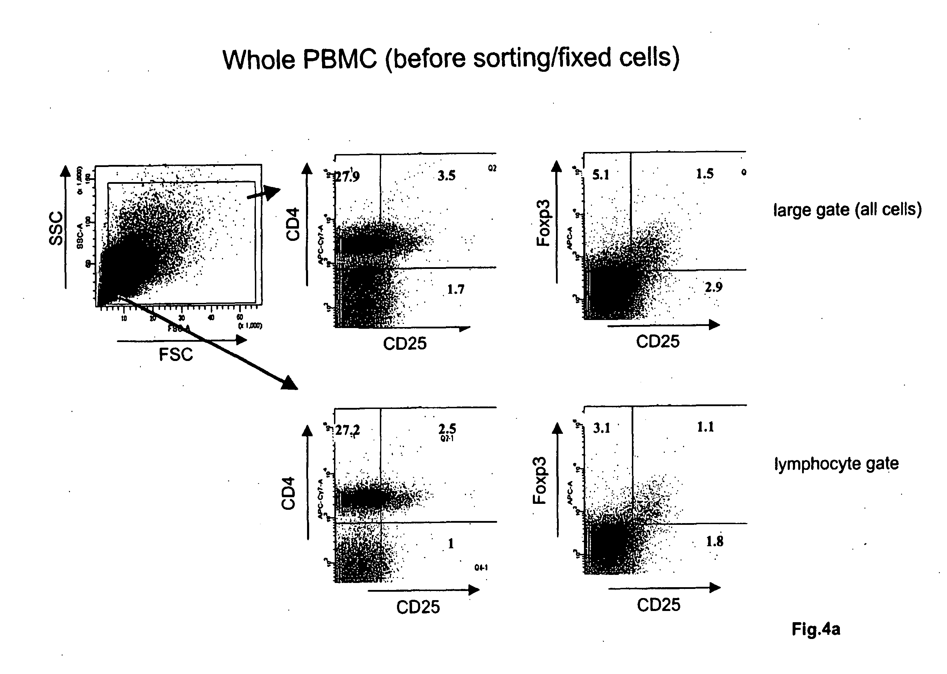

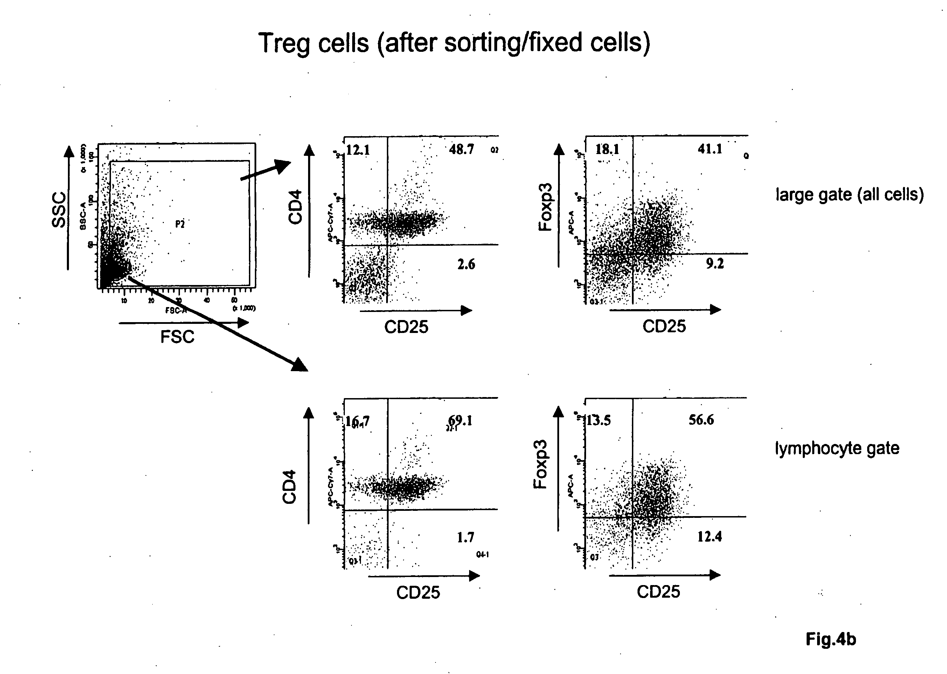

**Fig.4****:** Purity of Treg cells according to Foxp3 expression. The starting population and the obtained bead negative population after separation were surface stained with CD25 and CD4 and fixed for the subsequent analysis of intracellular Foxp3 expression. **a.** FACS profile for the starting population of PBMC after fixation and staining with Foxp3. Left panel: Profile for SSC vs. FSC. Right panels: FACS profiles for all cells (upper row) or lymphocytes (lower row) as indicated by the arrows for CD4 vs. CD25 and Foxp3 vs. CD25 according to the gates in the SSC vs. FSC profile (left panel). **b.** FACS profile of the bead negative population after depletion of CD127 and CD49d and fixation/Foxp3 staining. Left panel: FACS profile for cells like in Fig.4a. Right panels: FACS profile of gated cells (arrows) according to Fig.4a for all cells (upper row) or lymphocytes (lower row) for CD4 vs. CD25 and CD127 vs. CD49d. Numbers indicate the percentage of cells in the corresponding quadrant.

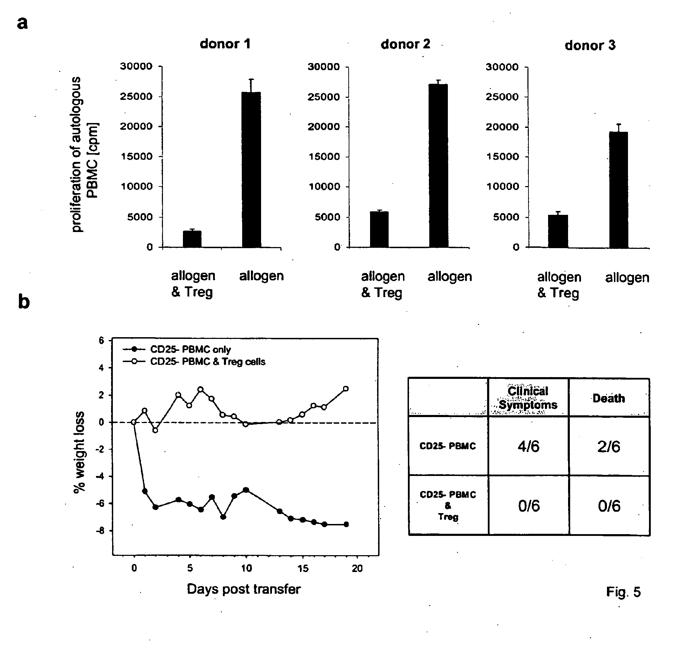

**Fig. 5****:** Inhibition of mixed lymphocyte reaction (MLR) *in vitro* and prevention of GvHD *in vivo*. Untouched Treg cells were isolated from human PBMC by a single-step CD127/CD49d-depletion as described in Fig. 4. **a.** Inhibition of MLR. MLR reaction is shown for three different donors. In each reaction 10⁵ PBMC were incubated with the same number of radiated PBMC of a haplotype-mismatched second donor (allogen). The reaction was inhibited by adding 2.5 x 10⁴ autologous Treg cells isolated in a single step MACS depletion (allogen & Treg). Proliferation was determined by ³H-thymidine incorporation and is expressed as 'counts per minute' (cpm). **b.** Prevention of acute GvHD. Acute xeno-GvHD was induced by the adoptive transfer of 30 x 10⁶ CD25 depleted human PBMC (CD25- PBMC) into Rag2 -/- γc -/- mice. Progression of the disease was recorded by determining the weight loss (left panel). One group received only CD25- PBMC (filled circle), a second group received CD25- PBMC together with 0.5 x 10⁶ untouched autologous Treg cells in a co-transfer (open circle). Groups of 6 mice were used; the average relative weight was determined in reference to the start of the experiment and is expressed as 'percent weight loss'. Incidence of clinical signs (ruffled fur, hunched posture and immobility) and death is indicated in the left panel.

The present invention shall now be described further in the following examples with respect to the attached figures without being limited thereto. Additionally, the present invention is based on scientific experiments which have been performed on biological specimen derived from patients. Patients have given their consent to use the specimen for the study which is disclosed in the present invention. In case of deceased patients, the consent has been given by a relative.

### Examples

Particular methods and materials used in the Examples:

### Antibodies and reagents

Antibodies specific for CD4 (RPA-T4), CD25 (MA251), and CD127 (hIL-7R-M21) were purchased from BD Bioscience. Anti-CD49d (BU49) was obtained from ImmunoTools. α-CD3 (UCHT-1) was produced at the MDC. αFoxp3 (PCH101) was purchased from eBioscience, intracellular staining was carried out according to manufacturer's recommendation. CFDA was obtained from Molecular Probes, ³H-Thymidine from GE Healthcare.

### Flow cytometry and cell preparation

Human PBMC were obtained from healthy volunteers. Mononuclear cells were isolated by Ficoll gradient centrifugation (GE Healthcare). FACS analysis was carried out on a FACSCalibur or LSR II instrument (BD Bioscience). Data were analysed using FACSDiva software (BD Bioscience), CellQuest (BD Bioscience) or Flowjo software (Treestar).

### Treg isolation

PBMC of a healthy donor were obtained by density gradient centrifugation (ficoll). 5x10⁷ cells were stained with αCD127-Biotin and αCD49d-Fitc for 10min. at 4-8°C. After washing with MACS buffer for 10min. at 4-8°C, cells were incubated with 20µl/107 cells of aFitc beads and 30µl/107 cells of aBiotin beads (Miltenyi Biotech) for 15min. at 4-8°C. After washing with MACS buffer for 10min. at 4-8°C, cells were depleted using a LD column (Miltenyi Biotech). After separation cells were stained with the indicated antibodies and analysed by FACS. For Foxp3 expression, cells were fixed and stained according to the manufacturer (eBioscience).

### CFDA-based proliferation assay

CD4+ effector cells were labelled with 0.5 µM 5-carboxyfluorescein diacetate (CFDA; Molecular Probes) as described before²⁵. CD4+ effector T cells (25.000 cells/well) were incubated with irradiated CD4-depleted PBMC (50.000 cells/well; 3000 rad) and 10 µg/ml anti-CD3 (UCHT-1) for 3-4 days in 96 well V-bottom plates (Costar). For T cell suppression Treg cells were added at the indicated ratio. Proliferation of CD4+ T cells was analyzed by FACS.

### Mixed lymphocyte reaction (MLR)

MLR with human PBMC was carried out as described before²⁶. In brief, PBMC (100.000 cells/well) were incubated with irradiated (3.000 rad) allogenic PBMC (100.000 cells/well) in RPMI/10% FCS (Invitrogen) for 5 days. For suppression of proliferation, isolated autologous Treg cells were added at the indicated ratio. Proliferation was monitored by ³H-Thymidine incorporation (1 µCi/well) for additional 10-15h of culture and determined using a beta-plate reader (Wallac).

### Xenogeneic-Graft versus Host disease (x-GvHD)

An acute form of GvHD was induced in Rag2 -/- γc -/- mice (purchased from Taconic) as described before⁹. In brief, PBMC were depleted with α-CD25 microbeads (Miltenyi Biotech) using a LD column (Miltenyi Biotech). Treg cells were obtained from PBMC of healthy donors using the one step procedure. One day before transfer, mice received i.v. 0.2 ml clodronate-containing liposomes. 4h prior to the transfer of cells, mice were irradiated (350rad). 30 x 10⁶ CD25 depleted PBMC were injected i.v., either alone or as mixture with 0.5 x 10⁵ Treg cells in PBS/0.1% human serum albumin. The weight of the mice and the clinical symptoms were determined over the entire period of the experiment; clinical signs of the disease were ruffled fur, hunched posture and impaired movement.

### Example 1

CD127 and CD49d are inversely correlated with Foxp3.
Depending on the donor, about 30-60 % of human PBMC are CD4+ T cells (Fig.1a), of these, approximately 3-10% are Treg cells. While human Treg cells express high levels of the IL-2 receptor α-chain CD25 (CD25^{high}), this marker does not separate the Treg population from non-regulatory CD4+ cells as clearly as in mice. Also some effector and memory CD4+ cells express lower amounts of CD25 (CD25^{low}), so that their population partly overlaps with the CD25^{high} Treg subset (Baecher-Allan, C., Brown, J. A., Freeman, G. J. & Hafler, D. A., J Immunol 167, 1245-53 (2001)). Treg separations based on this marker therefore have an inherent risk of being contaminated by these cells. Like several other genes, CD25 expression on Treg cells is driven directly by Foxp3 (Hori, S., Nomura, T. & Sakaguchi, S., Science 299, 1057-61 (2003)). Counterstaining with Foxp3 therefore indicates a near linear correlation for the CD25^{high} cells, in which cells expressing the highest amounts of Foxp3 also stain brightest for CD25 (Fig.1b, left panel).

In contrast to CD25, the α-chain of the IL-7 receptor (CD127) is inversely correlated with Foxp3 expression (Liu, W. et al., J Exp Med 203, 1701-11 (2006); Seddiki, N. et al., J Exp Med 203, 169.3-700 (2006)) (Fig. 1b, middle panel). Also here the correlation is nearly linear, but cells with highest level of Foxp3 have the lowest expression level of CD127. The segregation, however, is not complete. In the example shown in Fig. 1b (middle panel), about 2/3 of the CD127- cells were also Foxp3-. For the characterization of Treg cells CD127 is therefore always used in combination with CD25 (Liu, W. et al., J Exp Med 203, 1701-11 (2006); Seddiki, N. et al., J Exp Med 203, 1693-700 (2006)).

Our own studies have identified a second surface marker absent on most Treg cells. CD49d is the α-chain of the integrin VLA-4 (α₄β₁). Also here the co-segregation is incomplete (Fig. 1b, right panel). An inverse linear correlation with Foxp3, as observed for CD127, however, does apparently not exist.

Double-staining instead revealed absence of CD49d in Foxp3+ cells independent of the level of Foxp3 expression.

### Example 2

CD49d discriminates Foxp3+ Treg cells from Foxp3- CD127- cells.
While the segregation of both markers with Treg cells was incomplete, the combined use of CD127 and CD49d may complement each other. Double-staining with α-CD127 and α-CD49d allowed dividing the population of CD4+ cells into the three major populations: CD127+, CD49d+CD127- and CD49d-CD127- cells (Fig. 2a, upper panel). Staining with α-CD25 and α-Foxp3 confirmed that the vast majority of the CD127+ cells were non-regulatory CD25-Foxp3- cells (Fig. 2a, lower left panel). More importantly, CD49d divided the CD127- subset into two nearly equally large subpopulations. The majority of CD49d+CD127-cells was Foxp3-, only less than 18% were CD25+Foxp3+ (Fig. 2a, lower right panel). The CD49d-CD127- population, in contrast, consisted almost exclusively of Foxp3+ cells. More than 83% of the cells were CD25+Foxp3+ Treg which express high levels of Foxp3 (Fig. 2a, lower middle panel).

To confirm that the CD49d-CD127- phenotype correlates with suppressive capacity, CD49d+CD127- and CD49d-CD127- cells were isolated by FACS sorting from CD4+ PBMC (Fig. 2b). Almost no inhibition was observed with the CD49d+CD127- subset (Fig. 2c). In contrast, CD4+ cells sorted only based on the absence of CD49d and CD127 effectively prevented the expansion of activated CD25-CD4+ cells. Thus, the use of CD49d allows discriminating the non-suppressive CD127- cells from functional Foxp3+ Treg cells.

### Example 3

Purification of untouched human Treg cells by MACS.
The result of the FACS sorting experiment indicated that the combined depletion of CD4+ T cells with α-CD127 and α-CD49d by MACS should produce a clean population of untouched Treg cells. To validate applicability of this approach, human PBMC were labelled with α-CD127 and α-CD49d and depleted by MACS using conventional magnetic bead-labelled antibodies (Fig. 3 and Fig. 4).

### Example 4

Inhibition of allo-/xenogenic reactions *in vitro* and *in vivo*.
To confirm function and viability of Treg cells isolated by direct PBMC-depletion the suppressive capacity was tested in a mixed lymphocyte reaction (MLR). MLR is the in vitro correlate of GvHD. It is based on the allogenic activation of T cells by 'foreign' MHC molecules, evident after mixing PBMC of two different donors. To determine whether the Treg cells are able to control the alloreactive response, they were added to non-radiated autologous PBMC mixed with radiated PBMC of haplotype-mismatched donors (Fig. 5a). In these experiments, Treg cells purified from PBMC according to Example 3 proved to be potent suppressors of the allogenic response. As shown for three different donors, the presence of Treg cells strongly suppressed the proliferation of autologous PBMC. Thus, untouched PBMC-derived Foxp3+ cells are fully functional Treg cells able to control allogenic immune responses in vitro.

The MLR experiment clearly demonstrated the suppressive capacity of the isolated Treg cells in vitro. With regard to future therapeutic applications it is crucial, however, to demonstrate that untouched Treg cells can also suppress destructive immune responses in vivo. For this purpose an acute GvHD in vivo model was used, which is based on the transfer of CD25-depleted human PBMC into Rag2 -/- γc -/- mice (Mutis, T. et al., Clin Cancer Res 12, 5520-5 (2006)). The elimination of Treg cells from the transferred cell population allows simulating a particularly aggressive form of the disease which frequently leads to the death of the animals. Depletion of Treg cells from PBMC was carried out by MACS using α-CD25 microbeads, untouched Treg cells were isolated again in a single step by combined use of α-CD127/α-CD49d together with a commercial CD4+ isolation kit. All mice that received 30 x 10⁶ CD25-depleted PBMC exhibited a more or less pronounced weight loss within the first days of the experiment (Fig. 5b). 4 of the 6 mice developed clinical symptoms and of these 2 mice died during the experiment. In line with a previous publication the severity of PBMC-induced GvHD was diminished when autologous Treg cells were co-transferred (Mutis, T. et al., Clin Cancer Res 12, 5520-5 (2006)). In this case, 0.5 x 10⁶ untouched Treg cells were sufficient to completely abrogate the weight loss and all mice of the treated group remained without symptoms for the entire course of the experiment. Thus, untouched Treg cells isolated by CD127/CD49d-depletion are potent suppressor cells capable to control destructive immune responses both in vitro and in vivo.

### Industrial applicability

The method according to the present invention has the following advantages:
1.) Safety. The regulations for reagents employed in human therapy are very strict. This applies particularly for the compounds administered during the treatment. Cells produced by positive sorting are carrying a foreign antibody on their surface. The inherent risk of antibodies is difficult to determine, and also 'humanization' does not necessarily prevent adverse effects, as documented recently in the dramatic failure of super-agonistic α-CD28 (Suntharalingam, G. et al., N Engl J Med 355, 1018-28 (2006); Sharpe, A. H. & Abbas, A. K., N Engl J Med 355, 973-5 (2006)). This risk is avoided when untouched Treg cells are used that have been purified only with depleting antibodies.
2.) Viability and functional status of the cells. Another advantage untouched cells have compared to positively sorted cells is viability in vivo. Antibody-tagged cells are prone to depletion. Antibodies on the surface of cells can bind complement or, depending on the antibody subtype, attach to Fc-receptors on the surface of NK cells or macrophages. The outcome of this interaction is often cell death by complement- mediated lyses or 'antibody dependent cell-mediated cyotoxicity' (ADCC). Binding of antibody-coated beads may also lead to the formation of larger cell clusters that are eliminated by non-specific up-take mechanisms of the recipient. As a consequence a significant fraction of the transferred cells may get eliminated before they can exhibit a beneficial effect. Moreover, the binding of antibodies to cell surface receptors during purification may also alter the functional state. The molecule targeted during positive sorting of Treg cells is CD25. As α-chain of the IL-2 receptor it is vital for survival and function of Treg cells. Blocking may abrogate expansion and suppressor function (Thornton, A. M., Donovan, E. E., Piccirillo, C. A. & Shevach, E. M., J Immunol 172, 6519-23 (2004); Fontenot, J. D., Rasmussen, J. P., Gavin, M. A. & Rudensky, A. Y., Nat Immunol 6, 1142-51 (2005); Kohm, A. P. et al., J Immunol 176, 3301-5 (2006); McNeill, A., Spittle, E. & Backstrom, B. T., Scand J Immunol 65, 63-9 (2007)), while cross-linking of the IL-2 receptor by antibodies, on the other hand, can lead to a premature activation (Barnard, A. L., Igakura, T., Tanaka, Y., Taylor, G. P. & Bangham, C. R., Blood 106, 988-95 (2005); von Bonin, A., Huhn, J. & Fleischer, B., Immunol Rev 161, 43-53 (1998)), potential complications that can simply be avoided when using untouched Treg cells.
3.) Time & Costs. Compared to standard methods the new approach is cost effective and fast. The CD49d/CD127 can be adapted to all common separation methods. For example, the method can be performed using only a single MACS column. Thus, isolation does not require expensive equipment and is faster and easier than conventional MACS-based methods.
4.) Purity & GMP-compatibility. Flow cytometry based cell sorters are currently not approved for 'good manufacturing practice' (GMP). For clinical trials the isolation of donor cells has to be carried out by magnetic-bead technologies. First attempts to isolate Treg cells under GMP conditions, however, yielded an average purity of less than 50% (Hoffmann, P. et al., Biol Blood Marrow Transplant 12, 267-74 (2006)). Since the MACS purification was based on positive selection of CD25+ cells, the contaminating cells were mostly CD4+CD25^{low} effector and memory cells, posing an inherent risk to trigger GvHD when transferred into immune deficient patients. As a MACS-based method, the new approach is GMP compatible. Treg cells are obtained by a significantly higher degree of purity >70%, >80%, >90%, >95%, or even >98% with virtually no contaminating CD4+ effector cells. Moreover, the Treg cells are isolated untouched, which further improves compatibility of the procedure with GMP.

Accordingly, the method of the present invention offers a simple and cost effective way to isolate human Foxp3+ Treg cells. Additionally, the 'untouched' status of the cells, high purity and GMP compatibility of the method make the method widely applicable in different settings. The new method can therefore be employed to access 'untouched' human Treg cells for research & development, diagnosis and also offers a new perspective for their routine use for the manufacture of medicaments for immunotherapies.

### Cited Literature

Fontenot, J. D. & Rudensky, A. Y. A well adapted regulatory contrivance: regulatory T cell development and the forkhead family transcription factor Foxp3. Nat Immunol 6, 331-7 (2005) .
Sakaguchi, S. Naturally arising CD4+ regulatory t cells for immunologic self-tolerance and negative control of immune responses. Annu Rev Immunol 22, 531-62 (2004).
Baecher-Allan, C. & Hafler, D. A. Human regulatory T cells and their role in autoimmune disease. Immunol Rev 212, 203-16 (2006)
Robinson, D. S., Larche, M. & Durham, S. R. Tregs and allergic disease. J Clin Invest 114, 1389-97 (2004)
Roncarolo, M. G. & Battaglia, M. Regulatory T-cell immunotherapy for tolerance to self antigens and alloantigens in humans. Nat Rev Immunol 7, 585-98 (2007)
Hoffmann, P. et al. Isolation of CD4+CD25+ regulatory T cells for clinical trials. Biol Blood Marrow Transplant 12, 267-74 (2006)
Seddiki, N. et al. Expression of interleukin (IL)-2 and IL-7 receptors discriminates between human regulatory and activated T cells. J Exp Med 203, 1693-700 (2006)
Baecher-Allan, C., Brown, J. A., Freeman, G. J. & Hafler, D. A. CD4+CD25high regulatory cells in human peripheral blood. J Immunol 167, 1245-53 (2001).
Suntharalingam, G. et al. Cytokine storm in a phase 1 trial of the anti-CD28 monoclonal antibody TGN1412. N Engl J Med 355, 1018-28 (2006).
Liu, W. et al. CD127 expression inversely correlates with FoxP3 and suppressive function of human CD4+ T reg cells. J Exp Med 203, 1701-11 (2006).
Hori, S., Nomura, T. & Sakaguchi, S. Control of regulatory T cell development by the transcription factor Foxp3. Science 299, 1057-61 (2003)
Mutis, T. et al. Human regulatory T cells control xenogeneic graft-versus-host disease induced by autologous T cells in RAG2-/-gammac-/- immunodeficient mice. Clin Cancer Res 12, 5520-5 (2006)
Sharpe, A. H. & Abbas, A. K. T-cell costimulation--biology, therapeutic potential, and challenges. N Engl J Med 355, 973-5 (2006)
Thornton, A. M., Donovan, E. E., Piccirillo, C. A. & Shevach, E. M. Cutting edge: IL-2 is critically required for the in vitro activation of CD4+CD25+ T cell suppressor function. J Immunol 172, 6519-23 (2004).
Fontenot, J. D., Rasmussen, J. P., Gavin, M. A. & Rudensky, A. Y. A function for interleukin 2 in Foxp3-expressing regulatory T cells. Nat Immunol 6, 1142-51 (2005).
Kohm, A. P. et al. Cutting Edge: Anti-CD25 monoclonal antibody injection results in the functional inactivation, not depletion, of CD4+CD25+ T regulatory cells. J Immunol 176, 3301-5 (2006).
McNeill, A., Spittle, E. & Backstrom, B. T. Partial depletion of CD69low-expressing natural regulatory T cells with the anti-CD25 monoclonal antibody PC61. Scand J Immunol 65, 63-9 (2007).
Barnard, A. L., Igakura, T., Tanaka, Y., Taylor, G. P. & Bangham, C. R. Engagement of specific T-cell surface molecules regulates cytoskeletal polarization in HTLV-1-infected lymphocytes. Blood 106, 988-95 (2005).
von Bonin, A., Huhn, J. & Fleischer, B. Dipeptidyl-peptidase IV/CD26 on T cells: analysis of an alternative T-cell activation pathway. Immunol Rev 161, 43-53 (1998).
Randolph, D. A. & Fathman, C. G. Cd4+Cd25+ regulatory T cells and their therapeutic potential. Annu Rev Med 57, 381-402 (2006).
Tang, Q. & Bluestone, J. A. Regulatory T-cell physiology and application to treat autoimmunity. Immunol Rev 212, 217-37 (2006).
Masteller, E. L. et al. Expansion of functional endogenous antigen-specific CD4+CD25+ regulatory T cells from nonobese diabetic mice. J Immunol 175, 3053-9 (2005).
Waldmann, H. et al. Regulatory T cells and organ transplantation. Semin Immunol 16, 119-26 (2004).
Fontenot, J. D. et al. Regulatory T cell lineage specification by the forkhead transcription factor foxp3. Immunity 22, 329-41 (2005).
Kleinewietfeld, M. et al. CCR6 expression defines regulatory effector/memory-like cells within the CD25(+)CD4+ T-cell subset. Blood 105, 2877-86 (2005).
Ebert, L. M. & McColl, S. R. Coregulation of CXC chemokine receptor and CD4 expression on T lymphocytes during allogeneic activation. J Immunol 166, 4870-8 (2001).
P.T. Sharpe, Methods of Cell Separation, Laboratory Techniques in Biochemistry and Molecular Biology, Vol. 18, ELSEVIER (1988)
D. Fisher, G. E. Francis, D. Rickwood (Eds.) ,Cell Separation: A Practical Approach, Oxford University Press (1999)

## Claims

1. A method for isolating human Foxp3+ CD25+ regulatory T cells (Foxp3+ Treg cells) from a sample containing peripheral blood mononuclear cells (PBMCs), the method comprising the steps of:
(a) treating the sample with an anti-CD127 antibody and an anti-CD49d antibody;
(b) separating Foxp3+ Treg cells.

2. The method according to claim 1, wherein steps (a) and (b) are carried out simultaneously.

3. The method according to claim 1 or claim 2, wherein step (a) additionally comprises separation of non CD4+ T cells from PBMCs.

4. The method according to claim 3, wherein non CD4+ T cells are separated from PBMCs by negative selection using an anti-CD4 antibody.

5. The method according to claim 3, wherein one or more antibody/antibodies that allow for the specific depletion of non CD4+ T cells from PBMCs is/are used for the separation of non CD4+ T cells from PBMCs.

6. The method according to claim 5, wherein the anibody/antibodies used for the specific depletion of non CD4+ T cells from PBMCs is/are selected from the group comprising anti-CDS antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody, or mixtures.

7. The method according to claim 5 or claim 6, wherein anti-CD14 antibody, anti-CD15 antibody, and/or anti-CD16 antibody is/are used for the specific depletion of non CD4+ T cells from PBMCs

8. The method according to anyone of claims 5 to 7, wherein anti-CD14 antibody and/or anti-CD16 antibody is/are used for the specific depletion of non CD4+ T cells from PBMCs.

9. The method according to anyone of claims 1 to 8, wherein at least one of the antibodies used in step (a) is labelled or immobilized.

10. The method according to claim 9, wherein at least one antibody used in step (a) is immobilized.

11. The method according to claim 9 or claim 10, wherein at least one antibody used in step (a) is immobilized on a nylon matrix.

12. The method according to claim 9, wherein the antibodies used in step (a) are uniformly labelled.

13. The method according to claim 9 or claim 12, wherein the label is selected from the group comprising isotopes, fluorescence or luminescence markers, antibodies or antibody fragments, affinity labels, and enzymes or enzyme substrates.

14. The method according, to anyone of claims 1 to 9, 12 or 13, wherein the anti-CD127 antibody is labelled with biotin, fluorescein (FITC) or phycoerythrin (PE).

15. The method according to anyone of claims 1 to 9 and 12 to 14, wherein the anti-CD49d antibody is labelled with biotin, fluorescein (FITC) or phycoerythrin (PE).

16. The method according to anyone of claims 1 to 15, wherein step (b) is carried out using centrifugation, cell elutriation, magnetic separation, fluorescence activated cell sorting, immunological separation, adhesion, complement lysis or flow cytometry.

17. The method according to anyone of claims 1 to 16, wherein step (b) is carried out using magnetic cell separation, fluorescence activated cell sorting, or a column-based immunological separation.

18. The method according to anyone of claims 1 to 17, wherein an anti-CD45RO antibody is used as an additional antibody in step (a), and wherein the isolated Foxp3+ Treg cells are CD45RA+ T cells.

19. The method according to anyone of claims 1 to 17, wherein an anti-CD45RA antibody is used as an additional antibody in step (a), and wherein the isolated Foxp3+ Treg cells are CD45RO+ T cells.

20. A kit for isolating human Foxp3+ Treg cells, comprising an anti-CD49d antibody and an anti-CD127 antibody.

21. The kit according to claim 20, wherein the kit additionally comprises one or more antibody/antibodies selected from the group comprising anti-CD8 antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody.

22. The kit according to claim 20 or claim 21, wherein at least one of the antibodies is immobilized.

23. The kit according to claim 20 or claim 21, wherein at least one of the antibodies is labelled.

24. The kit according to claim 23, wherein the antibodies used are uniformly labelled.

25. The kit according to claim 23 or claim 2-4, wherein the label is selected from the group comprising isotopes, fluorescence or luminescence markers, antibodies or antibody fragments, affinity labels, and enzymes or enzyme substrates.

26. The kit according to anyone of claims 20, 21, 23, 24 or 25, wherein the anti-CD127 antibody is labelled with biotin, FITC, or PE and the anti-CD49d antibody is labelled with biotin, FITC, or PE.

27. Use of anti-CD49d antibody and anti-CD127 antibody or of a kit according to anyone of claims 20 to 26 for the isolation of human Foxp3+ Treg cells.

28. The use according to claim 27, **characterized in that** separation of the human Foxp3+ Treg cells is achieved by separating CD49d+CD127+ PBMCs including non-regulatory CD4+ T cells from unlabelled Foxp3+ Treg cells via centrifugation, cell elutriation, magnetic separation, fluorescence activated cell sorting, immunological separation, adhesion, complement lysis or flow cytometry.

29. The use according to claim 27 or claim 28, **characterized in that** depletion of non CD4+ T cells from PBMCs is carried out using at least one antibody selected from the group comprising anti-CD8 antibody, anti-CD14 antibody, anti-CD15 antibody, anti-CD16 antibody, anti-CD19 antibody, anti-CD36 antibody, anti-CD49b antibody, anti-CD56 antibody, anti-CD123 antibody, anti-TCRg/d antibody, and anti-CD235a antibody.

## Patentansprüche

1. Verfahren zur Isolierung von humanen regulatorischen Foxp3+ CD25+ T-Zellen (Foxp3+ Treg-Zellen) aus einer Probe, enthaltend periphere mononukleäre Blutzellen (peripheral blood mononuclear cells; PBMCs), wobei das Verfahren die Schritte umfasst:
(a) Behandeln der Probe mit einem anti-CD127 Antikörper und einem anti-CD49d Antikörper;
(b) Abtrennen der Foxp3+ Treg-Zellen.

2. Verfahren nach Anspruch 1, wobei die Schritte (a) und (b) simultan ausgeführt werden.

3. Verfahren nach Anspruch 1 oder Anspruch 2, wobei Schritt (a) zusätzlich das Abtrennen der nicht-CD4+ T-Zellen von den PBMCs umfasst.

4. Verfahren nach Anspruch 3, wobei die nicht-CD4+ T-Zellen von den PBMCs durch negative Selektion unter Verwendung eines anti-CD4 Antiköpers abgetrennt werden.

5. Verfahren nach Anspruch 3, wobei einer oder mehrere Antikörper, der/die die spezifische Depletion der nicht-CD4+ T-Zellen von den PBMCs ermöglicht/ermöglichen, für die Abtrennung der nicht-CD4+ T-Zellen von den PBMCs verwendet wird/werden.

6. Verfahren nach Anspruch 5, wobei der Antikörper/die Antikörper der/die für die spezifische Depletion der nicht-CD4+ T-Zellen von den PBMCs verwendet wird/werden, ausgewählt wird/werden aus der Gruppe, umfassend anti-CD8 Antikörper, anti-CD14 Antikörper, anti-CD15 Antikörper, anti-CD16 Antikörper, anti-CD19 Antikörper, anti-CD36 Antikörper, anti-CD49b Antikörper, anti-CD56 Antikörper, anti-CD123 Antikörper, anti-TCRγ/δ Antikörper, und anti-CD235a Antikörper, oder Mischungen.

7. Verfahren nach Anspruch 5 oder Anspruch 6, wobei anti-CD14 Antikörper, anti-CD15 Antikörper, und/oder anti-CD16 Antikörper für die spezifische Depletion der nicht-CD4+ T-Zellen von den PBMCs verwendet wird/werden.

8. Verfahren nach irgendeinem der Ansprüche 5 bis 7, wobei anti-CD14 Antikörper und/oder anti-CD16 Antikörper für die spezifische Depletion der nicht-CD4+ T-Zellen von den PBMCs verwendet wird/werden.

9. Verfahren nach irgendeinem der Ansprüche 1 bis 8, wobei mindestens einer der Antikörper, die in Schritt (a) verwendet werden, markiert oder immobilisiert ist.

10. Verfahren nach Anspruch 9, wobei mindestens ein in Schritt (a) verwendeter Antikörper immobilisiert ist.

11. Verfahren nach Anspruch 9 oder Anspruch 10, wobei mindestens ein in Schritt (a) verwendeter Antikörper an einer Nylon-Matrix immobilisiert ist.

12. Verfahren nach Anspruch 9, wobei die in Schritt (a) verwendeten Antikörper einheitlich markiert sind.

13. Verfahren nach Anspruch 9 oder Anspruch 12, wobei der Marker ausgewählt ist aus der Gruppe, umfassend Isotope, Fluoreszenz- oder Lumineszenz-Marker, Antikörper oder Antikörperfragmente, Affinitätsmarker, und Enzyme oder Enzymsubstrate.

14. Verfahren nach irgendeinem der Ansprüche 1 bis 9, 12 oder 13, wobei der anti-CD127 Antikörper mit Biotin, Fluorescein (FITC) oder Phycoerythrin (PE) markiert ist.

15. Verfahren nach irgendeinem der Ansprüche 1 bis 9, und 12 bis 14, wobei der anti-CD49d Antikörper mit Biotin, Fluorescein (FITC) oder Phycoerythrin (PE) markiert ist.

16. Verfahren nach irgendeinem der Ansprüche 1 bis 15, wobei Schritt (b) ausgeführt wird unter Verwendung von Zentrifugation, Zell-Elutrierung, magnetischer Abtrennung, Fluoreszenz aktivierter Zellsortierung, immunologischer Abtrennung, Adhäsion, komplementärer Lyse oder Durchflusszytometrie.

17. Verfahren nach irgendeinem der Ansprüche 1 bis 16, wobei Schritt (b) ausgeführt wird unter Verwendung von magnetischer Zellabtrennung, Fluoreszenz aktivierter Zellsortierung, oder einer Säulen-basierten immunologischen Abtrennung.

18. Verfahren nach irgendeinem der Ansprüche 1 bis 17, wobei ein anti-CD45RO Antikörper als zusätzlicher Antikörper in Schritt (a) verwendet wird, und wobei die isolierten Foxp3+ Treg-Zellen CD45RA+ T-Zellen sind.

19. Verfahren nach irgendeinem der Ansprüche 1 bis 17, wobei ein anti-CD45RA Antikörper als zusätzlicher Antikörper in Schritt (a) verwendet wird, und wobei die isolierten Foxp3+ Treg-Zellen CD45RO+ T-Zellen sind.

20. Kit zur Isolierung von humanen Foxp3+ Treg-Zellen, umfassend einen anti-CD49d Antikörper und einen anti-CD127 Antikörper.

21. Kit nach Anspruch 20, wobei der Kit zusätzlich einen oder mehrere Antikörper umfasst, ausgewählt aus der Gruppe, umfassend anti-CD8 Antikörper, anti-CD14 Antikörper, anti-CD15 Antikörper, anti-CD16 Antikörper, anti-CD19 Antikörper, anti-CD36 Antikörper, anti-CD49b Antikörper, anti-CD56 Antikörper, anti-CD123 Antikörper, anti-TCRγ/δ Antikörper, und anti-CD235a Antikörper.

22. Kit nach Anspruch 20 oder Anspruch 21, wobei mindestens einer der Antikörper immobilisiert ist.

23. Kit nach Anspruch 20 oder Anspruch 21, wobei mindestens einer der Antikörper markiert ist.

24. Kit nach Anspruch 23, wobei die verwendeten Antikörper einheitlich markiert sind.

25. Kit nach Anspruch 23 oder Anspruch 24, wobei der Marker ausgewählt ist aus der Gruppe, umfassend Isotope, Fluoreszenz- oder Lumineszenz-Marker, Antikörper oder Antikörperfragmente, Affinitätsmarker, und Enzyme oder Enzymsubstrate.

26. Kit nach irgendeinem der Ansprüche 20, 21, 23, 24 oder 25, wobei der anti-CD127 Antikörper mit Biotin, FITC, oder PE markiert ist und der anti-CD49d Antikörper mit Biotin, FITC, oder PE markiert ist.

27. Verwendung eines anti-CD49d Antikörpers und eines anti-CD127 Antikörpers, oder eines Kits nach irgendeinem der Ansprüche 20 bis 26 zur Isolierung humaner Foxp3+ Treg-Zellen.

28. Verwendung nach Anspruch 27, **dadurch gekennzeichnet, dass** die Abtrennung der humanen Foxp3+ Treg-Zellen erreicht wird, indem CD49d+CD127+ PBMCs einschließlich nicht-regulatorischer CD4+ T-Zellen von unmarkierten Foxp3+ Treg-Zellen mittels Zentrifugation, Zell-Elutrierung, magnetischer Abtrennung, Fluoreszenz aktivierter Zellsortierung, immunologischer Abtrennung, Adhäsion, komplementärer Lyse oder Durchflusszytometrie abgetrennt werden.

29. Verwendung nach Anspruch 27 oder Anspruch 28, **dadurch gekennzeichnet, dass** die Depletion der nicht-CD4+ T-Zellen von den PBMCs ausgeführt wird unter Verwendung von mindestens einem Antikörper, ausgewählt aus der Gruppe, umfassend anti-CD8 Antikörper, anti-CD14 Antikörper, anti-CD15 Antikörper, anti-CD16 Antikörper, anti-CD19 Antikörper, anti-CD36 Antikörper, anti-CD49b Antikörper, anti-CD56 Antikörper, anti-CD123 Antikörper, anti-TCRγ/δ Antikörper, und anti-CD235a Antikörper.

## Revendications

1. Procédé d'isolement de lymphocytes T régulateurs Foxp3+ CD25+ humains (cellules Foxp3+ Treg) d'un échantillon contenant des cellules mononucléaires de sang périphérique (PBMC), le procédé comprenant les étapes consistant à :
(a) traiter l'échantillon avec un anticorps anti-CD127 et un anticorps anti-CD49d ;
(b) séparer les cellules Foxp3+ Treg.

2. Procédé selon la revendication 1, dans lequel les étapes (a) et (b) sont réalisées simultanément.

3. Procédé selon la revendication 1 ou la revendication 2, dans lequel l'étape (a) comprend en outre la séparation de lymphocytes T non CD4+ des PBMC.

4. Procédé selon la revendication 3, dans lequel les lymphocytes T non CD4+ sont séparés des PBMC par sélection négative en utilisant un anticorps anti-CD4.

5. Procédé selon la revendication 3, dans lequel un ou plusieurs anticorps qui permettent la déplétion spécifique des lymphocytes T non CD4+ des PBMC est/sont utilisé(s) pour la séparation des lymphocytes T non CD4+ des PBMC.

6. Procédé selon la revendication 5, dans lequel le/les anticorps utilisés pour la déplétion spécifique des lymphocytes T non CD4+ des PBMC est/sont choisi(s) dans le groupe comprenant l'anticorps anti-CD8, l'anticorps anti-CD14, l'anticorps anti-CD15, l'anticorps anti-CD16, l'anticorps anti-CD19, l'anticorps anti-CD36, l'anticorps anti-CD49b, l'anticorps anti-CD56, l'anticorps anti-CD123, l'anticorps anti-TCRg/d et l'anticorps anti-CD235a ou leurs mélanges.

7. Procédé selon la revendication 5 ou la revendication 6, dans lequel l'anticorps anti-CD14, l'anticorps anti-CD15 et/ou l'anticorps anti-CD16 est/sont utilisé(s) pour la déplétion spécifique des lymphocytes T non CD4+ des PBMC.

8. Procédé selon l'une quelconque des revendications 5 à 7, dans lequel l'anticorps anti-CD14 et/ou l'anticorps anti-CD16 est/sont choisi(s) pour la déplétion spécifique des lymphocytes T non CD4+ des PBMC.

9. Procédé selon l'une quelconque des revendications 1 à 8, dans lequel au moins l'un des anticorps utilisés à l'étape (a) est marqué ou immobilisé.

10. Procédé selon la revendication 9, dans lequel au moins un anticorps utilisé à l'étape (a) est immobilisé.

11. Procédé selon la revendication 9 ou la revendication 10, dans lequel au moins un anticorps utilisé à l'étape (a) est immobilisé sur une matrice de nylon.

12. Procédé selon la revendication 9, dans lequel les anticorps utilisés à l'étape (a) sont marqués uniformément.

13. Procédé selon la revendication 9 ou la revendication 12, dans lequel le marqueur est choisi dans le groupe comprenant des isotopes, des marqueurs par fluorescence ou luminescence, des anticorps ou des fragments d'anticorps, des marqueurs d'affinité et des enzymes ou des substrats enzymatiques.

14. Procédé selon l'une quelconque des revendications 1 à 9, 12 ou 13, dans lequel l'anticorps anti-CD127 est marqué avec de la biotine, de la fluorescéine (FITC) ou de la phycroérythrine (PE).

15. Procédé selon l'une quelconque des revendications 1 à 9 et 12 à 14, dans lequel l'anticorps anti-CD49d est marqué avec de la biotine, de la fluorescéine (FITC) ou de la phycoérythrine (PE).

16. Procédé selon l'une quelconque des revendications 1 à 15, dans lequel l'étape (b) est réalisée en utilisant la centrifugation, l'élutriation de cellules, la séparation magnétique, le tri cellulaire activé par fluorescence, la séparation immunologique, l'adhérence, la lyse du complément ou la cytométrie de flux.

17. Procédé selon l'une quelconque des revendications 1 à 16, dans lequel l'étape (b) est réalisée en utilisant la séparation cellulaire magnétique, le tri cellulaire activé par fluorescence ou une séparation immunologique par colonne.

18. Procédé selon l'une quelconque des revendications 1 à 17, dans lequel un anticorps anti-CD45RO est utilisé comme anticorps supplémentaire à l'étape (a) et dans lequel les cellules Foxp3+ Treg isolées sont des lymphocytes T CD45RA+.

19. Procédé selon l'une quelconque des revendications 1 à 17, dans lequel un anticorps anti-CD45RA est utilisé comme anticorps supplémentaire à l'étape (a) et dans lequel les cellules Foxp3+ Treg isolées sont des lymphocytes T CD45RO+.

20. Kit pour l'isolement de cellules Foxp3+ Treg humaines, comprenant un anticorps anti-CD49d et un anticorps anti-CD127.

21. Kit selon la revendication 20, le kit comprenant en outre un ou plusieurs anticorps choisis dans le groupe comprenant l'anticorps anti-CD8, l'anticorps anti-CD14, l'anticorps anti-CD15, l'anticorps anti-CD16, l'anticorps anti-CD19, l'anticorps anti-CD36, l'anticorps anti-CD49b, l'anticorps anti-CD56, l'anticorps anti-CD123, l'anticorps anti-TCRg/d et l'anticorps anti-CD235a.

22. Kit selon la revendication 20 ou la revendication 21, dans lequel au moins l'un des anticorps est immobilisé.

23. Kit selon la revendication 20 ou la revendication 21, dans lequel au moins l'un des anticorps est marqué.

24. Kit selon la revendication 23, dans lequel les anticorps utilisés sont marqués uniformément.

25. Kit selon la revendication 23 ou la revendication 24, dans lequel le marqueur est choisi dans le groupe comprenant des isotopes, des marqueurs par fluorescence ou luminescence, des anticorps ou des fragments d'anticorps, des marqueurs d'affinité et des enzymes ou des substrats enzymatiques.

26. Kit selon l'une quelconque des revendications 20, 21, 23, 24 ou 25, dans lequel l'anticorps anti-CD127 est marqué avec de la biotine, du FITC ou du PE et l'anticorps anti-CD49d est marqué avec de la biotine, du FITC ou du PE.

27. Utilisation d'anticorps anti-CD49d et d'anticorps anti-CD127 ou d'un kit selon l'une quelconque des revendications 20 à 26 pour l'isolement de cellules Foxp3+ Treg humaines.

28. Utilisation selon la revendication 27, **caractérisée en ce que** la séparation des cellules Foxp3+ Treg humaines est réalisée par séparation des PBMC CD49d+CD127+ comprenant les lymphocytes T CD4+ non régulateurs de cellules Foxp3+ Treg non marquées par centrifugation, élutriation de cellules, séparation magnétique, tri cellulaire activé par fluorescence, séparation immunologique, adhérence, lyse du complément ou cytométrie de flux.

29. Utilisation selon la revendication 27 ou la revendication 28, **caractérisée en ce que** la déplétion des lymphocytes T non CD4+ des PBMC est réalisée en utilisant au moins un anticorps choisi dans le groupe comprenant l'anticorps anti-CD8, l'anticorps anti-CD14, l'anticorps anti-CD15, l'anticorps anti-CD16, l'anticorps anti-CD19, l'anticorps anti-CD36, l'anticorps anti-CD49b, l'anticorps anti-CD56, l'anticorps anti-CD123, l'anticorps anti-TCRg/d et l'anticorps anti-CD235a.
